# EUROPÄISCHE PATENTSCHRIFT

(11) **EP 2 869 814 B1**
(45) Veröffentlichungstag und Bekanntmachung des Hinweises auf die Patenterteilung: **06.04.2016**
(21) Anmeldenummer: 13735235.7
(22) Anmeldetag: 28.06.2013
(51) Int. Cl.: A61K 9/20, A61K 31/5377, A61K 9/00, A61K 9/24

(54) **PHARMAZEUTISCHE DARREICHUNGSFORMEN ENTHALTEND 5-CHLOR-N-({(5S)-2-OXO-3-[4-(3-OXO-4-MORPHOLINYL)-PHENYL]-1,3-OXAZOLIDIN-5-YL}-METHYL)-2-THIOPHEN-CARBOXAMID**
PHARMACEUTICAL ADMINISTRATION FORMS COMPRISING 5-CHLORO-N-({(5S)-2-OXO-3-[4-(3-OXO-4-MORPHOLINYL)PHENYL]-1,3-OXAZOLIDIN-5-YL}METHYL)-2-THIOPHENECARBOXAMIDE
FORMES PHARMACEUTIQUES CONTENANT DU 5-CHLORO-N-({(5S)-2-OXO-3-[4-(3-OXO-4-MORPHOLINYL)-PHÉNYL]-1,3-OXAZOLIDIN-5-YL}-MÉTHYL)-2-THIOPHÈNE-CARBOXAMIDE

(30) Priorität: 03.07.2012 EP 12174797
(43) Veröffentlichungstag der Anmeldung: 13.05.2015
(73) Patentinhaber: Bayer Pharma Aktiengesellschaft, 13353 Berlin (DE)
(72) Erfinder: BENKE, Klaus, 51465 Bergisch Gladbach (DE); NEUMANN, Heike, 42117 Wuppertal (DE); MÜCK, Wolfgang, 42113 Wuppertal (DE)
(74) Vertreter: BIP Patents
(86) Internationale Anmeldenummer: PCT/EP2013/063590
(87) Internationale Veröffentlichungsnummer: WO 2014/005934

(56) Entgegenhaltungen:
- EP-B1- 1 830 855
- WO-A2-2011/107750

## Beschreibung

Die vorliegende Erfindung betrifft feste, oral applizierbare pharmazeutische Darreichungsformen enthaltend 5-Chlor-*N*-({(5*S*)-2-oxo-3-[4-(3-oxo-4-morpholinyl)-phenyl]-1,3-oxazolidin-5-yl}-methyl)-2-thiophen-carboxamid (Rivaroxaban, Wirkstoff (I)), dadurch gekennzeichnet, dass eine Teilmenge des Wirkstoffs (I) schnell und eine Teilmenge kontrolliert (modifiziert, retardiert, verzögert) freigegeben wird, sowie Verfahren zu ihrer Herstellung, ihrer Anwendung als Arzneimittel, sowie ihrer Verwendung zur Prophylaxe, Sekundärprophylaxe oder Behandlung von Erkrankungen.

5-Chlor-N-({(5*S*)-2-oxo-3-[4-(3-oxo-4-morpholinyl)-phenyl]-1,3-oxazolidin-5-yl; -methyl)-2-thiophen-carboxamid (Rivaroxaban, Wirkstoff (I)) ist ein niedermolekularer, oral applizierbarer Inhibitor des Blutgerinnungsfaktors Xa, der zur Prophylaxe, Sekundärprophylaxe und/oder Behandlung verschiedener thromboembolischer Erkrankungen eingesetzt werden kann [WO O1/47919]. Wenn im Folgenden von Wirkstoff (I) die Rede ist, so ist dabei die Kristallmodifikation I von 5-Chlor-*N*-({(5*S*)-2-oxo-3-[4-(3-oxo-4-morpholinyl)-phenyl]-1,3-oxazolidin-5-yl}-methyl)-2-thiophen-carboxamid (I) gemeint.

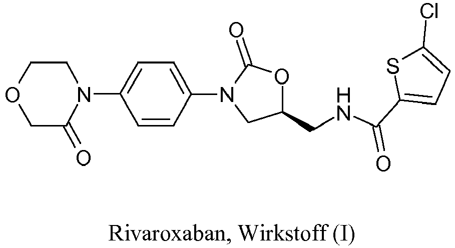

Bei Krankheiten, die über einen längeren Zeitraum behandelt werden müssen, oder zur längerfristigen Prophylaxe von Krankheiten ist es wünschenswert, die Häufigkeit der Einnahme von Medikamenten so gering wie möglich zu halten. Dies ist nicht nur bequemer für den Patienten, sondern es erhöht auch die Behandlungssicherheit, indem es die Nachteile unregelmäßiger Einnahmen vermindert (Verbesserung der Compliance). Die gewünschte Reduktion der Einnahmefrequenz, beispielsweise von zweimal täglicher auf einmal tägliche Applikation, kann über eine Verlängerung der therapeutisch effektiven Plasmaspiegel durch kontrollierte Wirkstofffreigabe aus den Darreichungsformen erreicht werden.

Nach Einnahme von Darreichungsformen mit kontrollierter Wirkstofffreisetzung kann außerdem durch Glättung des Plasmaspiegelverlaufs (Minimierung des so genannten peak-trough-Verhältnisses), also durch Vermeidung von hohen Plasmawirkstoffkonzentrationen, die häufig nach Gabe schnell freisetzender Arzneiformen zu beobachten sind, das Auftreten unerwünschter, mit den Konzentrationsspitzen korrelierter Nebenwirkungen vermindert werden.

Insbesondere für die Dauertherapie oder -prophylaxe und Sekundärprophylaxe arterieller und/oder venöser thromboembolischer Erkrankungen (beispielsweise tiefe Venenthrombosen, Schlaganfall, Myokardinfarkt und Lungenembolie) ist es von Vorteil, den Wirkstoff (I) in einer Form zur Verfügung zu haben, die über eine kontrollierte Wirkstofffreigabe zu einer Verringerung der Plasmaspitzen und damit zu einer Verringerung des peak-trough-Verhältnisses führt und eine einmal tägliche Applikation ermöglicht bei möglichst geringer Nahrungsmittelabhängigkeit der pharmakokinetischen Parameter (insbesondere cₘₐₓ und AUC) und möglichst hoher oraler Bioverfügbarkeit im Vergleich zu den Wirkstoff (I) schnell freisetzenden Formulierungen.

Bei der Formulierungsentwicklung sind die physikalisch-chemischen Charakteristika in Kombination mit den besonderen biologischen Eigenschaften des Wirkstoffs (I) zu berücksichtigen. Zu den physikalisch-chemischen Charakteristika zählt beispielsweise die geringe Wasserlöslichkeit des Wirkstoffs (I) (ca. 7 mg/l). Die besonderen biologischen Eigenschaften des Wirkstoffs (I) sind eine nahrungsmittelabhängige Bioverfügbarkeit (ein sogenannter Food-Effect), die bei schnell freisetzenden Tablettenformulierungen bei Dosierungen ab ca. 15 mg erkennbar wird, sowie eine eingeschränkte Absorption aus tieferen Darmabschnitten. Für die gewünschte einmal tägliche Applikation sind deshalb spezielle galenische Formulierungen notwendig, die den Wirkstoff (I) unter Berücksichtigung seiner biopharmazeutischen Charakteristika freisetzen, so dass der Wirkstoff (I) einerseits in für dessen pharmazeutische Wirkung ausreichender Menge enthalten ist und andererseits der Wirkstoff (I) so freigegeben wird, dass seine herausfordernden Eigenschaften betreffend Löslichkeit, Food-Effect und Absorption überwunden werden.

Wie in EP 1 830 855 B1 beschrieben, wurden zu diesem Zweck verschiedene Entwicklungsansätze verfolgt, darunter osmotische Zweikammersysteme. Charakterisiert wurden diese über ihr in-vitro Freisetzungsprofil und pharmakokinetische Studien in gesunden Probanden (n=12) im Vergleich zu einer den Wirkstoff (I) schnell freisetzenden Tablettenformulierung.

Die gemäß EP 1 830 855 B1 hergestellten und im Experimentellen Teil als Vergleichsformulierungen A bis C näher beschriebenen osmotischen Zweikammersysteme zeigen die gewünschten Glättungen der Blutplasmaprofile im Vergleich zu den schnell freisetzenden Tablettenformulierungen, weisen aber überraschenderweise eine ausgeprägte Nahrungsmittelabhängigkeit (Food-Effect) auf. Erfolgte die Applikation nach Gabe eines amerikanischen Frühstücks, erhöhte sich beispielsweise der maximal gemessene Blutplasmawert c_{max norm}) um den Faktor 2.08 bis 3.19 im Vergleich zur Applikation ohne Nahrung (Nüchterngabe). Dieser relativ stark ausgeprägte Food-Effect ist insbesondere für osmotische Systeme ungewöhnlich. So wird beispielsweise in "Biopharmazie - Pharmakokinetik - Bioverfügbarkeit - Biotransformation" (Langner/Borchert/Mehnert; Wissenschaftliche Verlagsgesellschaft Stuttgart, 4. Auflage, 2011, Seite 245) und in "Formulation-dependent food effects demonstrated for Nifedipine modified-release preparations marketed in the European Union" (Schug B.S., Brendel E., Wolf D., Wonnemann M., Wargenau M., Blume H.H.; European Journal of Pharmaceutical Sciences, 15, 2002, 279-285) auf die geringe Nahrungsmittelabhängigkeit der osmotischen Systeme als besonderer Vorteil hingewiesen.

Ziel der Entwicklung war es daher, eine geeignete Formulierung zu identifizieren, die im Vergleich zu einer den Wirkstoff (I) schnell freisetzenden Tablettenformulierung eine möglichst hohe relative Bioverfügbarkeit aufweist, im Vergleich zu den Wirkstoff (I) schnell freisetzenden Tablettenformulierungen geringere Plasmaspiegelspitzen erzeugt und eine einmal tägliche Gabe bei möglichst gering ausgeprägtem Nahrungsmitteleinfluss erlaubt.

Überraschenderweise wurde nun gefunden, dass Darreichungsformen, die einen Teil der Wirkstoffdosis schnell und einen Teil der Wirkstoffdosis mit kontrollierter (modifizierter, retardierter) Rate freisetzen, eine einmal tägliche Applikation mit geringer ausgeprägtem Nahrungsmitteleinfluss (Food-Effect) und besserer oraler Bioverfügbarkeit insbesondere bei Nüchterngabe erlauben.

Unter kontrollierter Freisetzung wird erfindungsgemäß eine Wirkstofffreisetzungscharakteristik verstanden, die nach der Einnahme bezüglich Zeit, Verlauf und/oder Ort im Magen-Darm-Trakt so eingestellt ist, wie sie nach Applikation konventioneller Formulierungen, wie z.B. oraler Lösungen oder den Wirkstoff schnell freisetzender fester Darreichungsformen wie beispielsweise in EP 1 689 370 B1 beschrieben, nicht erreicht werden kann. Neben dem Begriff "kontrollierte Freisetzung" werden häufig auch alternative Begriffe wie "modifizierte", "retardierte" oder "verzögerte" Freisetzung" verwendet. Diese sind vom Umfang der vorliegenden Erfindung ebenfalls mit umfasst.

Unter einer pharmazeutischen Darreichungsform mit kombinierter schneller und kontrollierter Freisetzung wird verstanden, dass die in der Darreichungsform enthaltene Wirkstoffdosis in mindestens zwei Teile, nämlich in mindestens eine schnell freisetzende Wirkstoffdosis und mindestens eine kontrolliert freisetzende Wirkstoffdosis, aufgeteilt ist, die in mindestens zwei unterschiedlichen Kompartimenten der Darreichungsform eingearbeitet sind.

Dadurch wird erreicht, dass 10 bis 45% des Wirkstoffs (I) (bezogen auf die deklarierte Gesamtmenge des Wirkstoffs (I)) innerhalb 1 Stunde, 40 bis 70% des Wirkstoffs (I) innerhalb von 4 Stunden und mindestens 80% des Wirkstoffs (I) innerhalb von 10 Stunden gemäß USP-Freisetzungsmethode mit Apparatur 2 (Paddle) bei 50 bis 100 Umdrehungen pro Minute (UpM), bevorzugt 75 UpM, in 900 ml eines geeigneten Freisetzungsmediums, bevorzugt eines Phosphat-Citrat-Puffers von pH 6,8, und unter Nutzung eines Sinkers gemäß der Japanischen Pharmakopöe freigesetzt werden.

Die Kombination eines schnell freisetzenden Wirkstoffanteils (IR = immediate release) mit einem kontrolliert freisetzenden Wirkstoffanteil (CR = controlled release) ist prinzipiell bekannt. Dies gilt auch für osmotische Zweikammersysteme.

In Connor, D.F. und Steingard, R.J., "New Formulations of Stimulants for Attention-Deficit Hyperactivity Disorder", CNS Drugs 18 (2004), 1011-1030 bzw. Coghill, D., Seth, S., "Osmotic, controlled-release methylphenidate for the treatment of ADHD", Expert Opinion Pharmacotherapy 7 (2006), 2119-2136 sind verschiedene Möglichkeiten der IR- und CR-Kombination beschrieben, unter anderem die Kombination von Pellets mit verschiedenen Freisetzungsraten (SODAS™) oder die OROS^{®}-Technologie (osmotische Systeme) kombiniert mit einem wirkstoffhaltigen Filmüberzug, aus dem ein Teil der Wirkstoffdosis schnell freigesetzt wird. Eine einmal tägliche Applikation wird in diesem Fall verfolgt, um eine sehr kurze Halbwertszeit des Wirkstoffs auszugleichen, Nebenwirkungen zu reduzieren sowie eine zweite Tabletteneinnahme während des Schulbesuches der betroffenen Kinder zu vermeiden. Die Kombination mit dem IR-Anteil zielt hier also auf den direkten Wirkungseintritt. Im Unterschied zu Wirkstoff (I) weisen die eingesetzten Substanzen eine gute Löslichkeit auf.

WO 1993/000071 beschreibt ein osmotisches System, welches mit einem wirkstoffhaltigen Film überzogen ist, um nach einer initialen Wirkstofffreigabe innerhalb der ersten 5 bis 15 Minuten nach Applikation die weitere Wirkstofffreigabe um 30 Minuten bis 4,5 Stunden (angestrebt werden mindestens 2 Stunden) zu verzögern. Fokus dieser Anwendung sind medizinische Fragestellungen, bei denen ein zirkadianer Rhythmus zu berücksichtigen ist, beispielsweise beim Auftreten von Herzinfarkten, deren Inzidenz in den frühen Morgenstunden am größten ist. Eine Einnahme der Arzneiform am Abend soll mit Hilfe des zweiten Wirkstoffschubs nach einer ausreichend langen lag time das Herzinfarktrisiko der frühen Morgenstunden reduzieren. Die Anwendung schließt daher insbesondere Calciumkanalblocker und hier im Besonderen Verapamilhydrochlorid ein. Im Unterschied zu Wirkstoff (I) ist die Wasserlöslichkeit von Verapamilhydrochlorid sehr gut.

Durch die vorliegende Erfindung ist die Bereitstellung einer stabilen pharmazeutischen Darreichungsform möglich, die den Wirkstoff (I) einerseits in für dessen pharmazeutische Wirkung ausreichender Menge enthält und andererseits den Wirkstoff (I) so freigibt, dass die herausfordernden biopharmazeutischen Eigenschaften des Wirkstoffs (I) betreffend Löslichkeit, Food-Effect und Absorption berücksichtigt werden. Dazu wird der Wirkstoff (I) aus der pharmazeutischen Darreichungsform sowohl schnell als auch kontrolliert (modifiziert, retardiert) freigegeben und ermöglicht damit nach einer einmal täglichen Applikation im Vergleich zu schnell freisetzenden Tablettenformulierungen gemäß EP 1 689 370 B1 verringerte Plasmaspiegelspitzen, im Vergleich zu osmotischen Zweikammersystemen ohne schnell freisetzenden Anteil gemäß EP 1 830 855 B1 weniger stark ausgeprägte Nahrungsmittelabhängigkeit der pharmakokinetischen Parameter wie insbesondere Plasmaspiegel-Maximalwerte (cₘₐₓ) und Bioverfügbarkeit (AUC) und zudem im Vergleich zu einer schnell freisetzenden Tablettenformulierung gemäß EP 1 689 370 B1 eine hohe relative Bioverfügbarkeit von bevorzugt mindestens 80% nach Nüchterngabe.

Der Wirkstoff (I) liegt in der Kristallmodifikation vor, in der der Wirkstoff (I) bei der Herstellung nach dem in WO 01/47919 unter Beispiel 44 beschriebenen Weg erhalten wird und die im Folgenden als Modifikation I bezeichnet wird.

Der Wirkstoff (I) liegt in den erfindungsgemäßen pharmazeutischen Darreichungsformen in kristalliner Form vor. In einer besonders bevorzugten Ausführungsform der vorliegenden Erfindung wird der Wirkstoff (I) in mikronisierter Form der Kristallmodifikation I eingesetzt. Hierbei besitzt der Wirkstoff (I) bevorzugt eine mittlere Partikelgröße X₅₀ kleiner 10 µm, insbesondere kleiner 8 µm, sowie einen X₉₀-Wert kleiner 20 µm, insbesondere kleiner 15 µm.

Die erfindungsgemäßen festen, oral applizierbaren pharmazeutischen Darreichungsformen enthaltend 5-Chlor-*N-*({(5*S*)-2-oxo-3-[4-(3-oxo-4-morpholinyl)-phenyl]-1,3-oxazolidin-5-yl}-methyl)-2-thiophen-carboxamid (I) sind dadurch gekennzeichnet, dass sie aus einer Kombination aus schneller und kontrollierter Freisetzung bestehen, wobei
- die kontrolliert freisetzende Wirkstoffdosis in ein osmotisches Freisetzungssystem, bevorzugt ein osmotisches Zweikammersystem, inkorporiert ist,
und
- das osmotische Freisetzungssystem mit einem den Wirkstoff (I) schnell freigebenden System kombiniert ist, bevorzugt einem wirkstoffhaltigen Filmüberzug oder einem wirkstoffhaltigen Pulver- oder Granulatmantel (Mantel-Kern-Tablette).

Die erfindungsgemäßen festen, oral applizierbaren pharmazeutischen Darreichungsformen enthaltend 5-Chlor-*N-*({(5*S*)-2-oxo-3-[4-(3-oxo-4-morpholinyl)-phenyl]-1,3-oxazolidin-5-yl}-methyl)-2-thiophen-carboxamid (I) sind dadurch gekennzeichnet, dass sie aus einer Kombination aus schneller und kontrollierter Freisetzung bestehen, wobei
aus der pharmazeutischen Darreichungsform
10 bis 45% des Wirkstoffs (I) der deklarierten Gesamtwirkstoffmenge nach 1 Stunde, 40 bis 70% des Wirkstoffs (I) nach 4 Stunden und mindestens 80% des Wirkstoffs (I) nach 10 Stunden gemäß USP-Freisetzungsmethode mit Apparatur 2 (Paddle) freigesetzt werden.

Die erfindungsgemäßen festen, oral applizierbaren pharmazeutischen Darreichungsformen enthaltend 5-Chlor-*N-*((5*S*)-2-oxo-3-[4-(3-oxo-4-morpholinyl)-phenyl]-1,3-oxazolidin-5-yl}-methyl)-2-thiophen-carboxamid (I) sind dadurch gekennzeichnet, dass sie aus einer Kombination aus schneller und kontrollierter Freisetzung bestehen, wobei
- die kontrolliert freisetzende Wirkstoffdosis in ein osmotisches Freisetzungssystem, bevorzugt ein osmotisches Zweikammersystem, inkorporiert ist, und 55 bis 90% der deklarierten Gesamtwirkstoffmenge enthält,
- das osmotische Freisetzungssystem mit einem den Wirkstoff (I) schnell freigebenden System kombiniert ist, bevorzugt einem wirkstoffhaltigen Filmüberzug oder einem wirkstoffhaltigen Pulver- oder Granulatmantel (Mantel-Kern-Tablette), und 10 bis 45% der deklarierten Gesamtwirkstoffmenge enthält,
und wobei aus der pharmazeutischen Darreichungsform
- 10 bis 45% des Wirkstoffs (I) der deklarierten Gesamtwirkstoffmenge nach 1 Stunde, 40 bis 70% des Wirkstoffs (I) nach 4 Stunden und mindestens 80% des Wirkstoffs (I) nach 10 Stunden gemäß USP-Freisetzungsmethode mit Apparatur 2 (Paddle) freigesetzt werden.

In den erfindungsgemäßen pharmazeutischen Darreichungsformen beträgt die Gesamtdosis des Wirkstoffs (I) 2,5 mg bis 30 mg, bevorzugt 5 mg bis 25 mg, besonders bevorzugt 5 mg, 6 mg, 10 mg, 12 mg, 15 mg, 20 mg oder 24 mg.

Die Gesamtwirkstoffmenge ist auf einen schnell freisetzenden Anteil der Wirkstoffdosis und einen kontrolliert freisetzenden Anteil der Wirkstoffdosis aufgeteilt. Der schnell freisetzende Anteil der Wirkstoffdosis beträgt 10 bis 45% des Wirkstoffs (I). Der kontrolliert freisetzende Anteil der Wirkstoffdosis beträgt 55 bis 90% des Wirkstoffs (I).

Geeignete Darreichungsformen, die es ermöglichen, den Wirkstoff (I) in einer Kombination aus schneller und kontrollierter Freisetzung abzugeben, basieren also auf einem osmotischen Freisetzungssystem in Kombination mit einer schnell freisetzenden wirkstoffhaltigen Hülle, entweder in Form eines wirkstoffhaltigen Filmüberzugs oder in Form eines den Wirkstoff (I) schnell freisetzenden Pulver- oder Granulatmantels (Mantel-Kern-Tablette):

### 1. Kontrollierte Freisetzung mit Osmotischem Freisetzungssystem

Zur Realisierung eines osmotischen Freisetzungssystems werden vorzugsweise Tabletten mit einer semipermeablen Membran umgeben, die mindestens eine Öffnung, bevorzugt eine Öffnung, aufweist. Die wasserdurchlässige Membran ist für die Komponenten des Kerns undurchlässig, erlaubt aber den Eintritt von Wasser von außen über Osmose in das System. Das eingedrungene Wasser setzt dann über den entstehenden osmotischen Druck den Wirkstoff (I) gelöst oder suspendiert aus der oder den Öffnung/en in der Membran frei. Die Gesamtwirkstofffreisetzung und die Freisetzungsrate können über die Dicke und Porosität der semipermeablen Membran, die Zusammensetzung des Kerns und die Anzahl und Größe der Öffnung/en gesteuert werden. Formulierungsaspekte, Anwendungsformen und Informationen zu Herstellverfahren sind u. a. in folgenden Publikationen beschrieben:
- Santus, G., Baker, R.W., "Osmotic drug delivery: a review of the patent literature", Journal of Controlled Release 35 (1995), 1-21
- Verma, R.K., Mishra, B., Garg, S., "Osmotically controlled oral drug delivery", Drug Development and Industrial Pharmacy 26 (2000), 695-708
- Verma, R.K., Krishna, D.M., Garg, S., "Formulation aspects in the development of osmotically controlled oral drug delivery systems", Journal of Controlled Release 79 (2002), 7-27
- Verma, R. K., Arora, S., Garg, S., "Osmotic Pumps in drug delivery", Critical Reviews in Therapeutic Drug Carrier Systems 21 (2004), 477-520
- Malaterre, V., Ogorka, J., Loggia, N., Gurny, R., "Oral osmotically driven systems: 30 years of development and clinical use", European Journal of Pharmaceutics and Biopharmaceutics 73 (2009), 311-323
- US 4,327,725, US 4,765,989.

Für den Wirkstoff (I) besonders geeignet sind Zweikammersysteme (push-pull-Systeme). Der Wirkstoff (I) liegt in dem osmotischen Zweikammersystem in kristalliner, bevorzugt mikronisierter Form vor. Ein Vorteil dieses osmotischen Zweikammersystems in Zusammenhang mit Wirkstoff (I) ist die über einen längeren Zeitraum einstellbare gleichmäßige Freisetzungsrate.

Beim osmotischen Zweikammersystem besteht der Kern aus zwei Schichten, einer Wirkstoffschicht und einer Osmoseschicht. Ein derartiges osmotisches Zweikammersystem ist beispielsweise in DE 34 17 113 C2 ausführlich beschrieben.

Die Wirkstoffschicht des osmotischen Zweikammersystems, die 55 bis 90% der Gesamtwirkstoffmenge enthält, ist bevorzugt zusammengesetzt aus
- 2 bis 25% Wirkstoff (I)
und
- 60 bis 95% von einem oder mehreren osmotisch aktiven Polymeren, bevorzugt Polyethylenoxid mittlerer Viskosität (40 bis 100 mPa*s; 5%ige wässrige Lösung, 25°C).

Die Osmoseschicht des osmotischen Zweikammersystems ist bervorzugt zusammengesetzt aus
- 40 bis 90% von einem oder mehreren osmotisch aktiven Polymeren, bervorzugt Polyethylenoxid hoher Viskosität (5000 bis 8000 mPa*s; 1%ige wässrige Lösung, 25°C)
und
- 10 bis 40% eines osmotisch aktiven Zusatzes.

Die Differenz zu 100% in den einzelnen Schichten des osmotischen Zweikammersystems (Wirkstoffschicht und Osmoseschicht) wird unabhängig voneinander jeweils durch einen oder mehrere zusätzliche Bestandteile in Form von pharmazeutisch üblichen Hilfsstoffen gebildet. Die Prozentangaben beziehen sich jeweils auf die Gesamtmasse der jeweiligen Kernschicht.

Die Wirkstoffschicht des osmotischen Zweikammersystems, die 55 bis 90% der Gesamtwirkstoffmenge enthält, ist besonders bevorzugt zusammengesetzt aus 5 bis 15% Wirkstoff (I).

Die Wirkstoffschicht des osmotischen Zweikammersystems ist besonders bevorzugt zusammengesetzt aus 75 bis 90% von einem oder mehreren osmotisch aktiven Polymeren, bevorzugt Polyethylenoxid mittlerer Viskosität (40 bis 100 mPa*s; 5%ige wässrige Lösung, 25°C).

Weiterer Gegenstand der vorliegenden Erfindung sind feste, oral applizierbare pharmazeutische Darreichungsformen dadurch gekennzeichnet, dass sie aus einer Kombination aus schneller und kontrollierter Freisetzung bestehen, wobei das 55 bis 90% des Wirkstoffs (I) enthaltende osmotische Zweikammersystem besteht aus
A) einer Wirkstoffschicht mit der Zusammensetzung
   - 2 bis 25% Wirkstoff (I),
   - 60 bis 95% von einem oder mehreren osmotisch aktiven Polymeren
B) einer Osmoseschicht mit der Zusammensetzung
   - 40 bis 90% eines oder mehrerer osmotisch aktiver Polymere,
   - 10 bis 40% eines osmotisch aktiven Zusatzes
   und
C) einer Hülle, bestehend aus einem wasserdurchlässigen, für die Komponenten des Kerns jedoch undurchlässigen Material mit mindestens einer Öffnung auf der Wirkstoffseite.

Im Kern des osmotischen Zweikammersystems können als osmotisch aktive Zusätze beispielsweise alle wasserlöslichen Stoffe verwendet werden, deren Verwendung in der Pharmazie unbedenklich ist, wie z.B. die in Pharmakopöen oder in "Hager" und "Remington Pharmaceutical Science" erwähnten wasserlöslichen Hilfsstoffe. Insbesondere können wasserlösliche Salze von anorganischen oder organischen Säuren oder nichtionische organische Stoffe mit großer Wasserlöslichkeit wie z.B. Kohlehydrate, insbesondere Zucker, Zuckeralkohole oder Aminosäuren verwendet werden. Erfindungsgemäß besonders bevorzugt wird Natriumchlorid verwendet.

Gegebenenfalls im Kern zusätzlich vorhandene hydrophile quellbare Polymere sind beispielsweise Hydroxypropylcellulose, Hydroxypropylmethylcellulose, Natriumcarboxymethylcellulose, Natriumcarboxymethylstärke, Polyacrylsäuren bzw. deren Salze.

Gegebenenfalls im Kern zusätzlich vorhandene pharmazeutisch übliche Hilfsstoffe sind beispielsweise Pufferstoffe wie Natriumbicarbonat, Bindemittel wie Hydroxypropylcellulose, Hydroxypropylmethylcellulose und/oder Polyvinylpyrrolidon, Schmiermittel wie Magnesiumstearat, Netzmittel wie Natriumlaurylsulfat, Fließregulierungsmittel wie hochdisperses Siliciumdioxid sowie ein Farbpigment wie Eisenoxid in einer der beiden Schichten zur farblichen Differenzierung von Wirkstoff- und Osmoseschicht.

Die semipermeable Membran des osmotischen Arzneimittelfreisetzungssystems besteht aus einem wasserdurchlässigen, für die Komponenten des Kerns undurchlässigen Material. Solche Membranmaterialien sind im Prinzip bekannt und beispielsweise beschrieben in der EP 1 024 793 B1, Seite 3-4, deren Offenbarung hiermit durch Bezugnahme eingeschlossen ist. Erfindungsgemäß bevorzugt werden als Membranmaterial Celluloseacetat oder Gemische von Celluloseacetat und Polyethylenglycol eingesetzt. Die semipermeable Membran enthält bevorzugt 5 bis 20% Porenbildner bezogen auf das Trockengewicht der semipermeablen Membran. Der Anteil der semipermeablen Membran (Trockengewicht) beträgt in der erfindungsgemäßen osmotischen Darreichungsform üblicherweise 2 bis 15%.

Weiterer Gegenstand der vorliegenden Erfindung ist ein Verfahren zur Herstellung des erfindungsgemäßen osmotischen Zweikammersystems, wobei die Komponenten der Wirkstoffschicht gemischt und vorzugsweise granuliert werden, die Komponenten der Osmoseschicht gemischt und vorzugsweise granuliert werden, wobei bei der Herstellung beider Schichten die trockene Granulation mittels Walzenkompaktierung bevorzugt ist, und anschließend beide Granulate auf einer Zweischichttablettenpresse zu einer Zweischichttablette verpresst werden. Der so entstandene Kern wird dann mit einer semipermeablen Membran beschichtet, die Hülle wird auf der Wirkstoffseite mit einer oder mehreren Öffnungen versehen.

Um die initiale Wirkstofffreigabe zu gewährleisten, muss eine Beschichtung bzw. Umhüllung des osmotischen Freisetzungssystems mit einem wirkstoffhaltigen Filmüberzug oder einem wirkstoffhaltigen Pulver- oder Granulatmantel erfolgen. Speziell bei osmotischen Wirkstofffreisetzungssystemen auf Celluloseacetat-Basis muss beim Aufbringen einer weiteren Schicht unabhängig davon, ob wirkstoffhaltiger Filmüberzug oder wirkstoffhaltiger Pulver- oder Granulatmantel, aufgrund der glatten und hydrophoben Oberfläche des osmotischen Systems ein Optimum zwischen homogener Wirkstoffverteilung in dem resultierenden Überzug (Filmüberzug oder Pulver- oder Granulatmantel), dessen Dicke und der Prozessdauer bzw. Stabilität gefunden werden.

### 2. Schnell freisetzende Wirkstoffhülle

### 2.1 Wirkstoff im Filmüberzug

Als filmbildende Polymere können für die Aufbringung von wirkstoffhaltigen Filmüberzügen Cellulosederivate, synthetische Polymere und Mischungen daraus verwendet werden. Als Cellulosederivate seien Methylcellulose, Hydroxymethylpropylcellulose, Hydroxypropylcellulose, Carboxymethylcellulose-Natrium, Hydroxyethylcellulose und Mischungen daraus genannt. Als synthetische Polymere seien Polyvinylpyrrolidon, Vinylpyrrolidon-Vinylacetat-Copolymer, Polyvinylalkohol (PVA), Polyvinylacetat, partiell hydrolysierter Polyvinylalkohol, Polyvinylalkohol-Polyethylenglykol (PEG)-Copolymere und Mischungen daraus genannt. Bevorzugte Filmbildner sind in diesem Fall Polyvinylalkohol, Polyvinylacetat, partiell hydrolysierter Polyvinylalkohol, Polyvinylalkohol-Polyethylenglykol-Copolymere und Mischungen daraus.

Der Filmüberzug kann weitere Hilfsstoffe enthalten wie beispielsweise Netzmittel (z.B. Natriumsalze von Fettalkoholsulfaten wie Natriumlaurylsulfat, Sulfosuccinate wie Natriumdioctylsulfosuccinat, partielle Fettsäureester mehrwertiger Alkohole wie Glycerolmonostearat oder partielle Fettsäureester des Sorbitans wie Sorbitanmonolaurat), Pigmente (z. B. Titandioxid, Talkum), Farbpigmente (z. B. Eisenoxid rot, gelb oder schwarz oder Mischungen daraus), Trennmittel (z. B. Kaolin, Talkum, hochdisperses Siliciumdioxid, Magnesiumstearat, Glycerolmonostearat), und/oder Weichmacher (z. B. Polyethylenglykol, Polypropylenglycol, Propylenglycol, Glycerol, Triacetin, Triethylcitrat).

Ebenfalls einsetzbar sind kommerziell erhältliche Präparate, sogenannte Fertiglacke, die bereits weitere pharmazeutische Hilfsstoffe enthalten und einfach in Wasser dispergiert werden können. Beispielsweise seien genannt:
- Kollicoat IR Fertiglacke (BASF; PVA-co-PEG-basiert), bestehend aus Kollicoat IR, Kollidon VA64, Kaolin, Natriumlaurylsulfat und ggf. anderen Farbpigmenten und
- Opadry II Lacke (Colorcon; PVA-basiert), bestehend aus PVA (partiell hydrolysiert), Talkum, Polyethylenglykol, Titandioxid sowie ggf. Eisenoxiden und Lecithin.

Im wirkstoffhaltigen Filmüberzug beträgt der Anteil des Wirkstoffs (I) bevorzugt 5 bis 30%, besonders bevorzugt 10 bis 25%, und der Anteil an Netzmittel bevorzugt 0,1 bis 2%, besonders bevorzugt 0,25 bis 1%, bezogen auf das Trockengewicht des Filmüberzugs. Der Anteil des Filmüberzugs in der erfindungsgemäßen Darreichungsform ist bevorzugt im Bereich 5 bis 15%.

### 2.2. Pulver- oder Granulathülle als Mantelüberzug (Mantel-Kern-Tablette)

Der Mantelüberzug (=Pulver- bzw. Granulatmantel) muss, bedingt durch die ungünstige Oberflächenbeschaffenheit des membranlackierten osmotischen Freigabesystems, ausreichend dimensioniert sein, um eine ausreichende Stabilität des Mantels zu gewährleisten. Der den Wirkstoff (I) schnell freisetzende Pulver- bzw. Granulatmantel enthält die üblichen, dem Fachmann bekannten Hilfsstoffe, beispielsweise Netzmittel in Form von Tensiden (z.B. Natriumlaurylsulfat, Polysorbate), Bindemittel (z.B. Zucker, Zuckeralkohole, Stärken, Cellulosederivate, Alginate, Pektine, Polyethylenglykole oder Polyvilylpyrrolidon), Füllmittel in Form von Cellulosederivaten (z.B. mikrokristalline Cellulose), Stärken (nativ oder modifiziert, z.B. Kartoffelstärke), Zuckern (z.B. Laktose), Zuckeralkoholen (z.B. Mannit, Sorbit) sowie anorganischen Füllstoffen (z.B. Calciumphosphat, Magnesiumoxid), Sprengmittel in Form von Stärkederivaten (z.B. quervernetzte Natriumcarboxymethylstärke, Natriumstärkeglykolat), Cellulosederivaten (z.B. quervernetzte Carboxymethylcellulose) oder quervernetztes Polyvinylpyrrolidon, und Gleitmittel (hier als Oberbegriff für Schmier-/Gleit-/Fließmittel), z.B. Magnesiumstearat, Calciumstearat, Stearinsäure, Talkum und hochdisperses Siliciumdioxid.

Die Herstellung der erfindungsgemäßen Mantelhülle erfolgt nach dem Fachmann bekannten Methoden, vorzugsweise wie in EP 1 689 370 B1 beschrieben durch Wirbelschichtgranulation. Die den Wirkstoff (I) schnell freisetzenden Granulate werden anschließend als Hüllgranulat eingesetzt und mit dem osmotischen Zweikammersystem als Kern auf einer Mantel-Kern-Tablettenpresse zu einer Mantel-Kern-Tablette verpresst.

Im Mantelüberzug beträgt der Anteil des Wirkstoffs (I) bevorzugt 0,5 bis 10%, besonders bevorzugt 1 bis 5%, bezogen auf das Gewicht des Pulver- oder Granulatmantels. Ein Netzmittel kann optional bis zu 1% zugegeben werden und der Anteil des Pulver- oder Granulatmantels an der Gesamtmasse der erfindungsgemäßen Darreichungsform beträgt bevorzugt mindestens 50%.

Weiterer Gegenstand der vorliegenden Erfindung sind orale, einmal täglich applizierbare Arzneimittel, enthaltend eine erfindungsgemäße Darreichungsform.

Weiterer Gegenstand der vorliegenden Erfindung ist ein Verfahren zur Herstellung der erfindungsgemäßen festen, oral applizierbaren pharmazeutischen Darreichungsform mit kombinierter schneller und kontrollierter Freisetzung, dadurch gekennzeichnet, dass die Komponenten der Wirkstoffschicht gemischt und vorzugsweise granuliert werden, die Komponenten der Osmoseschicht gemischt und vorzugsweise granuliert werden, anschließend beide Granulate auf einer Zweischichttablettenpresse zu einer Zweischichttablette verpresst werden und der so entstandene Kern dann mit einer semipermeablen Membran beschichtet und die Hülle auf der Wirkstoffseite mit einer oder mehreren Öffnungen versehen wird und anschließend der so entstandene membranlackierte Kern mit einer schnell freisetzenden Wirkstoffschicht umgeben wird, indem entweder ein den Wirkstoff (I) enthaltender Filmüberzug aufgetragen wird oder ein mittels Wirbelschichtgranulation hergestelltes wirkstoffhaltiges Granulat um den Kern gepresst wird.

Weiterer Gegenstand der vorliegenden Erfindung sind Arzneimittel, enthaltend eine erfindungsgemäße feste, oral applizierbare, den Wirkstoff (I) enthaltende pharmazeutische Darreichungsform mit kombinierter schneller und kontrollierter (modifizierter, retardierter) Freisetzung des Wirkstoffs (I).

Weiterer Gegenstand der vorliegenden Erfindung ist die Verwendung einer festen, oral applizierbaren, den Wirkstoff (I) enthaltenden pharmazeutischen Darreichungsform mit kombinierter schneller und kontrollierter Freisetzung, zur Herstellung eines Arzneimittels zur Prophylaxe, Sekundärprophylaxe und/oder Behandlung von Erkrankungen, insbesondere von arteriellen und/oder venösen thromboembolischen Erkrankungen.

Weiterer Gegenstand der vorliegenden Erfindung ist die Verwendung der erfindungsgemäßen festen, oral applizierbaren pharmazeutischen Darreichungsform mit kombinierter schneller und kontrollierter Freisetzung, enthaltend den Wirkstoff (I) zur Prophylaxe, Sekundärprophylaxe und/oder Behandlung von Erkrankungen, insbesondere von arteriellen und/oder venösen thromboembolischen Erkrankungen wie Myocardinfarkt, Angina Pectoris (eingeschlossen instabile Angina), Reokklusionen und Restenosen nach einer Angioplastie oder aortokoronarem Bypass, Schlaganfall, transitorische ischämische Attacken, periphere arterielle Verschlusskrankheiten, Lungenembolien oder tiefen Venenthrombosen.

Weiterer Gegenstand der vorliegenden Erfindung ist die Verwendung der erfindungsgemäßen festen, oral applizierbaren, den Wirkstoff (I) enthaltende pharmazeutischen Darreichungsform mit kombinierter schneller und kontrollierter Freisetzung zur Herstellung eines Arzneimittels zur Prophylaxe, Sekundärprophylaxe und/oder Behandlung von Erkrankungen, insbesondere von arteriellen und/oder venösen thromboembolischen Erkrankungen wie Myocardinfarkt, Angina Pectoris (eingeschlossen instabile Angina), Reokklusionen und Restenosen nach einer Angioplastie oder aortokoronarem Bypass, Schlaganfall, transitorische ischämische Attacken, periphere arterielle Verschlusskrankheiten, Lungenembolien oder tiefen Venenthrombosen.

Weiterer Gegenstand der vorliegenden Erfindung ist die Verwendung von 5-Chlor-*N-*({(5*S*)-2-oxo-3-[4-(3-oxo-4-morpholinyl)-phenyl]-1,3-oxazolidin-5-yl}-methyl)-2-thiophen-carboxamid (I) zur Herstellung einer erfindungsgemäßen festen, oral applizierbaren pharmazeutischen Darreichungsform mit kombinierter schneller und kontrollierter Freisetzung.

Weiterer Gegenstand der vorliegenden Erfindung ist ein Verfahren zur Prophylaxe, Sekundärprophylaxe und/oder Behandlung von arteriellen und/oder venösen thromboembolischen Erkrankungen durch Verabreichung einer erfindungsgemäßen festen, oral applizierbaren, den Wirkstoff (I) enthaltende pharmazeutischen Darreichungsform mit kombinierter schneller und kontrollierter Freisetzung.

Die Erfindung wird nachstehend durch bevorzugte Ausführungsbeispiele näher erläutert, auf welche sie jedoch nicht eingeschränkt ist. Soweit nicht anders angegeben, beziehen sich nachstehend alle Mengenangaben auf Gewichtsprozente.

### Experimenteller Teil:

Soweit nicht anders angegeben, werden die im Folgenden beschriebenen in vitro Freisetzungsuntersuchungen gemäß USP-Freisetzungsmethode mit Apparatur 2 (Paddle) durchgeführt. Die Umdrehungsgeschwindigkeit des Rührers liegt bei 75 UpM (Umdrehungen pro Minute) in 900 ml eines Phosphat-Citrat-Puffers von pH 6,8, die hergestellt wird aus 1,25 ml ortho Phosphorsäure, 4,75 g Citronensäuremonohydrat und 27,47 g Dinatriumhydrogenphosphatdihydrat in 10 1 Wasser. Zur Einstellung von sink-Bedingungen wird der Lösung Natriumlaurylsulfat zugegeben. In Abhängigkeit von der Dosierung variiert die zugesetzte Menge, bevorzugt beträgt die zugesetzte Menge 0,05 bis 0,5% Natriumlaurylsulfat, besonders bevorzugt 0,2 bis 0,4% Natriumlaurylsulfat. Bevorzugt werden bei einer Wirkstoffdosis von 5 bis 10 mg Rivaroxaban 0.2% Natriumlaurylsulfat, bei einer Dosis von 11 bis 15 mg Rivaroxaban 0,3% Natriumlaurylsulfat und bei einer Dosis von 16 bis 24 mg Rivaroxaban 0.4% Natriumlaurylsulfat zugesetzt. Der Fachmann weiß, dass eine Mindestmenge an Natriumlaurylsulfat abhängig von der Dosis des Wirkstoffs benötigt wird, um sink-Bedingungen zu erreichen. Bei einer weiteren Erhöhung der Menge an Natriumlaurylsulfat über diese Mindestmenge hinaus verändern sich die Freisetzungsprofile jedoch nicht wesentlich. Die Freisetzung der Tabletten erfolgt aus einem Sinker gemäß der Japanischen Pharmakopöe.

**Beispielformulierung 1 (deklarierter Gehalt = 12 mg/Tablette)**

| **Kern** | |
|---|---|
| Wirkstoffschicht: | |
| Wirkstoff (I), mikronisiert | 8.4 mg |
| Hydroxypropylmethylcellulose (5 cp) | 5.7 mg |
| Polyethylenoxid * | 94.8 mg |
| Hochdisperses Siliziumdioxid (Aerosil 200) | 0.9 mg |
| Magnesiumstearat | 0.3 mg |
| | 110.1 mg |

| Osmoseschicht: | |
|---|---|
| Hydroxypropylmethylcellulose (5 cp) | 3.69 mg |
| Natriumchlorid | 21.51 mg |
| Polyethylenoxid ** | 47.60 mg |
| Eisenoxid rot | 0.72 mg |
| Magnesiumstearat | 0.18 mg |
| | 73.70 mg |

| **Membranüberzug** | |
|---|---|
| Celluloseacetat | 17.16 mg |
| Polyethylenglycol 3350 | 2.28 mg |
| | 19.44 mg |

| **Filmüberzug** | |
|---|---|
| Wirkstoff (I), mikronisiert | 4.0 mg |
| Natriumlaurylsulfat | 0.1 mg |
| Opadry II 85G35294 pink *** | 15.9 mg |
| | 20.0 mg |

| | |
|---|---|
| * Viskosität 5%ige wässrige Lösung (25°C, Brookfield Viskosimeter Modell RVT, Spindel Nr. 1, Drehzahl: 50 U/min): 40-100 mPa·s (z. B. POLYOX™ Water-Soluble Resin NF WSR N-80; Dow) ** Viskosität 1%ige wässrige Lösung (25°C, Brookfield Viskosimeter Modell RVF, Spindel Nr. 2, Drehzahl: 2 U/min): 5000-8000 mPa·s (z. B. POLYOX™ Water-Soluble Resin NF WSR Coagulant; Dow) *** Polyvinylalkohol (partiell hydrolysiert), Talkum, Polyethylenoxid 3350, Lecithin, Titandioxid, Eisenoxide | |

### Herstellung:

Die Komponenten der Wirkstoffschicht werden gemischt und trocken granuliert (Walzengranulation). Ebenso werden die Komponenten der Osmoseschicht gemischt und trocken granuliert (Walzengranulation). Auf einer Zweischichttablettenpresse werden beide Granulate zu einer Zweischichttablette (Durchmesser 8 mm) verpresst. Die Tabletten werden mit einer acetonischen Lösung von Celluloseacetat und Polyethylenglycol beschichtet und getrocknet. Anschließend wird bei jeder Tablette auf der Wirkstoffseite eine Öffnung von 0.9 mm Durchmesser mittels eines Handbohrers angebracht. Zur anschließenden Lackierung der Tabletten wird eine Suspension hergestellt, wobei zunächst Natriumlaurylsulfat in Wasser gelöst, hierauf der Wirkstoff (I) in dieser Lösung suspendiert und danach der Opadry-Fertiglack in dieser Suspension dispergiert wird.

**In vitro Freisetzung der Beispielformulierung 1**

| | | | | | | |
|---|---|---|---|---|---|---|
| **Zeit [min]** | 60 | 120 | 240 | 360 | 480 | 600 |
| **Freisetzung [%]** | 33 | 43 | 64 | 85 | 100 | 102 |

| | | | | | | |
|---|---|---|---|---|---|---|
| (USP-Paddle, 75 UpM, 900 ml Phosphatpuffer pH 6.8 + 0.3% NaLS, JP-sinker) | | | | | | |

**Beispielformulierung 2 (deklarierter Gehalt = 14 mg/Tablette)**

| **Kern** | |
|---|---|
| Wirkstoffschicht: | |
| Wirkstoff (I), mikronisiert | 8.4 mg |
| Hydroxypropylmethylcellulose (5 cp) | 5.7 mg |
| Polyethylenoxid * | 94.8 mg |
| Hochdisperses Siliziumdioxid (Aerosil 200) | 0.9 mg |
| Magnesiumstearat | 0.3 mg |
| | 110.1 mg |

| Osmoseschicht: | |
|---|---|
| Hydroxypropylmethylcellulose (5 cp) | 3.69 mg |
| Natriumchlorid | 21.51 mg |
| Polyethylenoxid ** | 47.60 mg |
| Eisenoxid rot | 0.72 mg |
| Magnesiumstearat | 0.18 mg |
| | 73.70 mg |

| **Membranüberzug** | |
|---|---|
| Celluloseacetat | 22.5 mg |
| Polyethylenglycol 3350 | 2.25 mg |
| | 24.75 mg |

| **Mantelüberzug** | |
|---|---|
| Wirkstoff (I), mikronisiert | 6.0 mg |
| Hydroxypropylmethylcellulose, 5 cP | 14.4 mg |
| Natriumlaurylsulfat | 0.9 mg |
| Mikrokristalline Cellulose | 192.0 mg |
| Laktose Monohydrat | 177.4 mg |
| Croscarmellose Natrium | 14.4 mg |
| Magnesiumstearat | 2.9 mg |
| | 408.0 mg |

| | |
|---|---|
| * Viskosität 5%ige wässrige Lösung (25°C, Brookfield Viskosimeter Modell RVT, Spindel Nr. 1, Drehzahl: 50 U/min): 40-100 mPa·s (z. B. POLYOX™ Water-Soluble Resin NF WSR N-80; Dow) ** Viskosität 1%ige wässrige Lösung (25°C, Brookfield Viskosimeter Modell RVF, Spindel Nr. 2, Drehzahl: 2 U/min): 5000-8000 mPa·s (z. B. POLYOX™ Water-Soluble Resin NF WSR Coagulant; Dow) | |

### Herstellung:

Kern: Die Komponenten der Wirkstoffschicht werden gemischt und trocken granuliert (Walzengranulation). Ebenso werden die Komponenten der Osmoseschicht gemischt und trocken granuliert (Walzengranulation). Auf einer Zweischichttablettenpresse werden beide Granulate zu einer Zweischichttablette (Durchmesser 8 mm) verpresst.

Membranüberzug: Die Tabletten werden mit einer acetonischen Lösung von Celluloseacetat und Polyethylenglycol beschichtet und getrocknet. Anschließend wird bei jeder Tablette auf der Wirkstoffseite eine Öffnung von 0.9 mm Durchmesser mittels eines Handbohrers angebracht.

Mantelüberzug: Hydroxypropylmethylcellulose, 5 cP und Natriumlaurylsulfat werden in Wasser gelöst. In diese Lösung wird der mikronisierte Wirkstoff (I) suspendiert. Die so hergestellte Suspension wird als Granulierflüssigkeit im Rahmen einer Wirbelschichtgranulation auf die Vorlage aus mikrokristalliner Cellulose, Laktose Monohydrat und Croscarmellose Natrium aufgesprüht. Nach Trocknung und Siebung (0.8 mm Maschenweite) des entstandenen Granulates wird Magnesiumstearat zugegeben und gemischt.

Das so erhaltene Granulat und die bereits membranüberzogenen Kerne werden an einer Mantel-Kern-Tablettenpresse zu Mantel-Kern-Tabletten (Durchmesser 12 mm) verpresst.

**In vitro Freisetzung der Beispielformulierung 2**

| | | | | | | |
|---|---|---|---|---|---|---|
| **Zeit [min]** | 60 | 120 | 240 | 360 | 480 | 600 |
| **Freisetzung [%]** | 44 | 47 | 61 | 75 | 88 | 98 |

| | | | | | | |
|---|---|---|---|---|---|---|
| (USP-Paddle, 75 UpM, 900 ml Phosphatpuffer pH 6.8 + 0.3% NaLS, JP-sinker) | | | | | | |

**Beispielformulierung 3 (deklarierter Gehalt = 12 mg/Tablette)**

| **Kern** | |
|---|---|
| Wirkstoffschicht: | |
| Wirkstoff (I), mikronisiert | 10.0 mg |
| Hydroxypropylmethylcellulose (5 cp) | 5.7 mg |
| Polyethylenoxid * | 93.2 mg |
| Hochdisperses Siliziumdioxid (Aerosil 200) | 0.9 mg |
| Magnesiumstearat | 0.3 mg |
| | 110.1 mg |

| Osmoseschicht: | |
|---|---|
| Hydroxypropylmethylcellulose (5 cp) | 3.69 mg |
| Natriumchlorid | 21.51 mg |
| Polyethylenoxid ** | 47.60 mg |
| Eisenoxid rot | 0.72 mg |
| Magnesiumstearat | 0.18 mg |
| | 73.70 mg |

| **Membranüberzug** | |
|---|---|
| Celluloseacetat | 14.58 mg |
| Polyethylenglycol 3350 | 1.94 mg |
| | 16.52 mg |

| **Filmüberzug** | |
|---|---|
| Wirkstoff (I), mikronisiert | 2.5 mg |
| Natriumlaurylsulfat | 0.1 mg |
| Opadry II 85G35294 pink *** | 17.4 mg |
| | 20.0 mg |

| | |
|---|---|
| * Viskosität 5%ige wässrige Lösung (25°C, Brookfield Viskosimeter Modell RVT, Spindel Nr. 1, Drehzahl: 50 U/min): 40-100 mPa·s (z. B. POLYOX™ Water-Soluble Resin NF WSR N-80; Dow) ** Viskosität 1%ige wässrige Lösung (25°C, Brookfield Viskosimeter Modell RVF, Spindel Nr. 2, Drehzahl: 2 U/min): 5000-8000 mPa·s (z. B. POLYOX™ Water-Soluble Resin NF WSR Coagulant; Dow) *** Polyvinylalkohol (partiell hydrolysiert), Talkum, Polyethylenoxid 3350, Lecithin, Titandioxid, Eisenoxide | |

Die Herstellung erfolgt analog der Beispielformulierung 1.

**In vitro Freisetzung der Beipielformulierung 3**

| | | | | | | |
|---|---|---|---|---|---|---|
| **Zeit [min]** | 60 | 120 | 240 | 360 | 480 | 600 |
| **Freisetzung [%]** | 19 | 32 | 59 | 85 | 96 | 96 |

| | | | | | | |
|---|---|---|---|---|---|---|
| (USP-Paddle, 75 UpM, 900 ml Phosphatpuffer pH 6.8 + 0.3% NaLS, JP-sinker) | | | | | | |

**Beispielformulierung 4 (deklarierter Gehalt = 12 mg/Tablette)**

| **Kern** | |
|---|---|
| Wirkstoffschicht: | |
| Wirkstoff (I), mikronisiert | 10.0 mg |
| Hydroxypropylmethylcellulose (5 cp) | 5.7 mg |
| Polyethylenoxid * | 93.2 mg |
| Hochdisperses Siliziumdioxid (Aerosil 200) | 0.9 mg |
| Magnesiumstearat | 0.3 mg |
| | 110.1 mg |

| Osmoseschicht: | |
|---|---|
| Hydroxypropylmethylcellulose (5 cp) | 3.69 mg |
| Natriumchlorid | 21.51 mg |
| Polyethylenoxid ** | 47.60 mg |
| Eisenoxid rot | 0.72 mg |
| Magnesiumstearat | 0.18 mg |
| | 73.70 mg |

| **Membranüberzug** | |
|---|---|
| Celluloseacetat | 17.16 mg |
| Polyethylenglycol 3350 | 2.28 mg |
| | 19.44 mg |

| **Filmüberzug** | |
|---|---|
| Wirkstoff (I), mikronisiert | 2.5 mg |
| Natriumlaurylsulfat | 0.1 mg |
| Opadry II 85G35294 pink *** | 17.4 mg |
| | 20.0 mg |

| | |
|---|---|
| * Viskosität 5%ige wässrige Lösung (25°C, Brookfield Viskosimeter Modell RVT, Spindel Nr. 1, Drehzahl: 50 U/min): 40-100 mPa·s (z. B. POLYOX™ Water-Soluble Resin NF WSR N-80; Dow) ** Viskosität 1%ige wässrige Lösung (25°C, Brookfield Viskosimeter Modell RVF, Spindel Nr. 2, Drehzahl: 2 U/min): 5000-8000 mPa·s (z. B. POLYOX™ Water-Soluble Resin NF WSR Coagulant; Dow) *** Polyvinylalkohol (partiell hydrolysiert), Talkum, Polyethylenoxid 3350, Lecithin, Titandioxid, Eisenoxide | |

Die Herstellung erfolgt analog der Beispielformulierung 1.

**In vitro Freisetzung der Beipielformulierung 4**

| | | | | | | |
|---|---|---|---|---|---|---|
| **Zeit [min]** | 60 | 120 | 240 | 360 | 480 | 600 |
| **Freisetzung [%]** | 19 | 27 | 50 | 72 | 91 | 96 |

| | | | | | | |
|---|---|---|---|---|---|---|
| (USP-Paddle, 75 UpM, 900 ml Phosphatpuffer pH 6.8 + 0.3% NaLS, JP-sinker) | | | | | | |

**Beispielformulierung 5 (deklarierter Gehalt = 12 mg/Tablette)**

| **Kern** | |
|---|---|
| Wirkstoffschicht: | |
| Wirkstoff (I), mikronisiert | 10.0 mg |
| Hydroxypropylmethylcellulose (5 cp) | 5.7 mg |
| Polyethylenoxid * | 93.2 mg |
| Hochdisperses Siliziumdioxid (Aerosil 200) | 0.9 mg |
| Magnesiumstearat | 0.3 mg |
| | 110.1 mg |

| Osmoseschicht: | |
|---|---|
| Hydroxypropylmethylcellulose (5 cp) | 3.69 mg |
| Natriumchlorid | 21.51 mg |
| Polyethylenoxid ** | 47.60 mg |
| Eisenoxid rot | 0.72 mg |
| Magnesiumstearat | 0.18mg |
| | 73.70 mg |

| **Membranüberzug** | |
|---|---|
| Celluloseacetat | 19.73 mg |
| Polyethylenglycol 3350 | 2.62 mg |
| | 22.35 mg |

| **Filmüberzug** | |
|---|---|
| Wirkstoff (I), mikronisiert | 2.5 mg |
| Natriumlaurylsulfat | 0.1 mg |
| Opadry II 85G35294 pink *** | 17.4 mg |
| | 20.0 mg |

| | |
|---|---|
| * Viskosität 5%ige wässrige Lösung (25°C, Brookfield Viskosimeter Modell RVT, Spindel Nr. 1, Drehzahl: 50 U/min): 40-100 mPa·s (z. B. POLYOX™ Water-Soluble Resin NF WSR N-80; Dow) ** Viskosität 1%ige wässrige Lösung (25°C, Brookfield Viskosimeter Modell RVF, Spindel Nr. 2, Drehzahl: 2 U/min): 5000-8000 mPa·s (z. B. POLYOX™ Water-Soluble Resin NF WSR Coagulant; Dow) *** Polyvinylalkohol (partiell hydrolysiert), Talkum, Polyethylenoxid 3350, Lecithin, Titandioxid, Eisenoxide | |

Die Herstellung erfolgt analog der Beispielformulierung 1.

**In vitro Freisetzung der Beipielformulierung 5**

| | | | | | | |
|---|---|---|---|---|---|---|
| **Zeit [min]** | 60 | 120 | 240 | 360 | 480 | 600 |
| **Freisetzung [%]** | 18 | 23 | 42 | 61 | 80 | 93 |

| | | | | | | |
|---|---|---|---|---|---|---|
| (USP-Paddle, 75 UpM, 900 ml Phosphatpuffer pH 6.8 + 0.3% NaLS, JP-sinker) | | | | | | |

### In vivo Ergebnisse: Humane Pharmakokinetikstudien

Die erfindungsgemäße Beispielformulierung 1 wurde in einer pharmakokinetischen Studie in gesunden Probanden (n = 11) crossover im Vergleich zu einer den Wirkstoff (I) schnell freisetzenden Tablettenformulierung getestet (Formulierung ist beschrieben als Beispiel 5.1 / Tablette B in EP 1 689 370 B1), wobei Beispielformulierung 1 nüchtern und nach Verabreichung eines Amerikanischen Frühstücks verabreicht wurde, um die Nahrungsmittelabhängigkeit der Bioverfügbarkeit (AUC) und der Plasmaspiegelspitzen (cₘₐₓ-Werte) zu überprüfen. Als relevante pharmakokinetische Parameter sind in Tabelle 1 die AUCₙₒᵣₘ- und c_{max norm}-Verhältniswerte angegeben (Verhältnis Beispielformulierung 1 (CR = controlled release) zur schnell freisetzenden Tablettenformulierung (IR = immediate release)) jeweils nüchtern (fasted) und nach Gabe eines amerikanischen Frühstücks (fed) sowie das Verhältnis dieser Werte.

**Tabelle 1: Beispielformulierung 1 im Vergleich zu einer schnell freisetzende Tablette 10 mg**

| **AUC norm Ratio** | |
|---|---|
| CR fasted / IR fasted | 0.870 |
| CR fed / IR fasted | 1.015 |
| CR fed / CR fasted | 1.17 |

| **Cmax norm Ratio** | |
|---|---|
| CR fasted / IR fasted | 0.582 |
| CR fed / IR fasted | 0.879 |
| CR fed / CR fasted | 1.51 |

| | |
|---|---|
| CR = controlled release = Beispielformulierung 1; IR = immediate release Tablette | |

Im Vergleich dazu sind die Ergebnisse aus analog durchgeführten pharmakokinetischen Studien mit den Vergleichsformulierungen A bis C tabellarisch aufgeführt. Diese osmotischen Zweikammersysteme ohne schnell freisetzenden Wirkstoffanteil haben folgende Zusammensetzung (Angaben in mg/Tablette):

| **Vergleichsformulierung** | **A** | **B** | **C** |
|---|---|---|---|
| **Deklarierter Gehalt** (mg/Tablette) | 10 | 20 | 12 |
| **Kern** | | | |

| ***Wirkstoffschicht*** | | | |
|---|---|---|---|
| Wirkstoff (I), mikronisiert | 11.0 | 22.0 | 12.5 |
| Hydroxypropylmethylcellulose (5 cP) | 9.1 | 8.5 | 5.7 |
| Polyethylenoxid * | 138.1 | 127.7 | 90.7 |
| Hochdisperses Siliciumdioxid (Aerosil 200, Degussa) | 1.3 | 1.3 | 0.9 |
| Magnesiumstearat | 0.6 | 0.6 | 0.3 |
| | 160.1 | 160.1 | 110.1 |

| ***Osmoseschicht*** | | | |
|---|---|---|---|
| Hydroxypropylmethylcellulose (5 cP) | 4.1 | 4.1 | 3.69 |
| Natriumchlorid | 23.9 | 23.9 | 21.51 |
| Polyethylenoxid** | 52.9 | 52.9 | 47.6 |
| Eisenoxid rot | 0.8 | 0.8 | 0.72 |
| Magnesiumstearat | 0.2 | 0.2 | 0.18 |
| | 81.9 | 81.9 | 73.7 |

| **Osmotische Membran** | | | |
|---|---|---|---|
| Celluloseacetat | 18.0 | 18.0 | 14.3 |
| Polyethylenglykol 400 | 3.0 | 3.0 | --- |
| Polyethylenglykol 3350 | --- | --- | 1.9 |
| | 21.0 | 21.0 | 16.2 |
| **Nicht-funktionaler Farblack** | 6.0 | 6.0 | --- |

| | | | |
|---|---|---|---|
| * Viskosität 5%ige wässrige Lösung (25°C, Brookfield Viskosimeter Modell RVT, Spindel Nr. 1, Drehzahl: 50 U/min): 40-100 mPa·s (z. B. POLYOX™ Water-Soluble Resin NF WSR N-80; Dow) ** Viskosität 1%ige wässrige Lösung (25°C, Brookfield Viskosimeter Modell RVF, Spindel Nr. 2, Drehzahl: 2 U/min): 5000-8000 mPa·s (z. B. POLYOX™ Water-Soluble Resin NF WSR Coagulant; Dow) | | | |

Die Herstellung der Tabletten A bis C erfolgte analog Beispielformulierung 3.2 aus EP 1 830 855 B1. Gewählte Tablettenformate waren für die Vergleichs-Formulierungen A und B 8.7 mm; Vergleichs-Formulierung C wurde zu 8 mm-Tabletten verpresst.

In vitro Freisetzungsraten [%] der Vergleichsformulierungen A bis C:

| **Zeit [min]** | **60** | **120** | **240** | **360** | **480** |
|---|---|---|---|---|---|
| **Formulierung A** | 4 | 20 | 56 | 86 | 101 |
| **Formulierung B** | 4 | 21 | 58 | 89 | 102 |
| **Formulierung C** | 1 | 16 | 52 | 87 | 99 |

| | | | | | |
|---|---|---|---|---|---|
| (USP Paddle, 75 UpM, 900 ml Phosphatpuffer pH 6.8 + NaLS, JP-sinker) | | | | | |

Die relevanten pharmakokinetischen Parameter der AUCₙₒᵣₘ- und c_{max norm}-Verhältniswerte sind in Tabelle 2 jeweils nüchtern (fasted) und nach Gabe eines Amerikanischen Frühstücks (fed) aufgeführt (Verhältnis Vergleichsformulierungen A, B und C (CR) zur schnell freisetzenden Tablettenformulierung (IR) sowie das Verhältnis dieser Werte).

**Tabelle 2: Vergleichsformulierungen A bis C im Vergleich zu schnell freisetzenden Tabletten 10 mg**

| | **A** | **B** | **C** |
|---|---|---|---|
| **AUC norm Ratio** | | | |
| CR fasted / IR fasted | 0.730 | 0.669 | 0.615 |
| CR fed / IR fasted | 0.961 | 0.944 | 0.989 |
| CR fed / CR fasted | 1.32 | 1.41 | 1.61 |

| **Cmax norm Ratio** | | | |
|---|---|---|---|
| CR fasted / IR fasted | 0.265 | 0.354 | 0.220 |
| CR fed / IR fasted | 0.646 | 0.736 | 0.702 |
| CR fed / CR fasted | 2.41 | 2.08 | 3.19 |

| | | | |
|---|---|---|---|
| CR = controlled release = Vergleichsformulierungen A bis C; IR = immediate release Tablette | | | |

Die Überlegenheit der Beispielformulierung 1 im Vergleich zu den Formulierungen A bis C ist damit gezeigt. Während die Vergleichsformulierungen A bis C c_{max norm}-Faktoren fed/fasted (also nach Gabe eines Amerikanischen Frühstücks im Vergleich zur Nüchterngabe) von 2.08 bis 3.19 aufweisen, liegt dieser Wert für die Beispielformulierung 1 nur bei 1.51 - die Nahrungsmittelabhängigkeit der c_{max norm}-Werte ist also deutlich weniger stark ausgeprägt.

Die Nahrungsmittelabhängigkeit der relativen Bioverfügbarkeit bei Beispielformulierung 1 ist ebenfalls weniger stark ausgeprägt (Faktor 1.17 im Vergleich zu 1.32 bis 1.61 bei Vergleichsformulierungen A bis C). Weiterhin konnte die relative Bioverfügbarkeit nach Nüchterngabe für Beispielformulierung 1 auf 87% im Vergleich zu 61,5 bis 73% der Vergleichsformulierungen A bis C erhöht werden.

## Patentansprüche

1. Feste, oral applizierbare pharmazeutische Darreichungsform enthaltend 5-Chlor-*N*-({(5*S*)-2-oxo-3-[4-(3-oxo-4-morpholinyl)-phenyl]-1,3-oxazolidin-5-yl}-methyl)-2-thiophen-carboxamid (I) **dadurch gekennzeichnet, dass** sie aus einer Kombination aus schneller und kontrollierter Freisetzung besteht, wobei
die kontrolliert freisetzende Wirkstoffdosis in ein osmotisches Zweikammersystem inkorporiert ist,
und
das osmotische Freisetzungssystem mit einem den Wirkstoff (I) schnell freigebenden wirkstoffhaltigen Filmüberzug oder einem wirkstoffhaltigen Pulver- oder Granulatmantel (Mantel-Kern-Tablette) kombiniert ist.

2. Feste, oral applizierbare pharmazeutische Darreichungsform gemäß Anspruch 1, **dadurch gekennzeichnet, dass** 55 bis 90% des Wirkstoffs (I) als kontrolliert freisetzender Anteil der Wirkstoffdosis in das osmotische Zweikammersystem inkorporiert sind, und 10 bis 45% des Wirkstoffs (I) als schnell freisetzender Anteil der Wirkstoffdosis in den schnell freigebenden wirkstoffhaltigen Filmüberzug oder einem wirkstoffhaltigen Pulver- oder Granulatmantel (Mantel-Kern-Tablette) inkorporiert sind.

3. Feste, oral applizierbare pharmazeutische Darreichungsform gemäß Anspruch 1 oder 2, **dadurch gekennzeichnet, dass** das 55 bis 90% des Wirkstoffs (I) enthaltende osmotische Zweikammersystem besteht aus
A) einer Wirkstoffschicht mit der Zusammensetzung
• 2 bis 25% Wirkstoff (I),
• 60 bis 95% von einem oder mehreren osmotisch aktiven Polymeren
B) einer Osmoseschicht mit der Zusammensetzung
• 40 bis 90% eines oder mehrerer osmotisch aktiver Polymere,
• 10 bis 40% eines osmotisch aktiven Zusatzes
und
C) einer Hülle, bestehend aus einem wasserdurchlässigen, für die Komponenten des Kerns jedoch undurchlässigen Material mit mindestens einer Öffnung auf der Wirkstoffseite.

4. Feste, oral applizierbare pharmazeutische Darreichungsform gemäß Anspruch 1 oder 2, **dadurch gekennzeichnet, dass** der 10 bis 45% des Wirkstoffs (I) enthaltende wirkstoffhaltige Filmüberzug zusammengesetzt ist aus 5 bis 30% des Wirkstoffs (I) und 0,1 bis 2% Netzmittel, bezogen auf das Trockengewicht des Filmüberzugs.

5. Feste, oral applizierbare pharmazeutische Darreichungsform gemäß Anspruch 1 oder 2, **dadurch gekennzeichnet, dass** der 10 bis 45% des Wirkstoffs (I) enthaltende wirkstoffhaltige Pulver- oder Granulatmantel (Mantel-Kern-Tablette) zusammengesetzt ist aus 0,5 bis 10% Wirkstoff (I) bezogen auf das Gewicht des Pulver- oder Granulatmantels.

6. Feste, oral applizierbare pharmazeutische Darreichungsform gemäß einem der Ansprüche 1 bis 5, **dadurch gekennzeichnet, dass** aus der pharmazeutischen Darreichungsform 10 bis 45% des Wirkstoffs (I) der deklarierten Gesamtwirkstoffmenge nach 1 Stunde, 40 bis 70% des Wirkstoffs (I) nach 4 Stunden und mindestens 80% des Wirkstoffs (I) nach 10 Stunden gemäß USP-Freisetzungsmethode mit Apparatur 2 (Paddle) freigesetzt werden.

7. Feste, oral applizierbare pharmazeutische Darreichungsform gemäß Anspruch 6, **dadurch gekennzeichnet, dass** die USP-Freisetzungsmethode mit Apparatur 2 (Paddle) bei 75 UpM in 900 ml eines Phosphat-Citrat-Puffers von pH 6,8 mit einem Zusatz von 0,4% Natriumlaurylsulfat als Freisetzungsmedium und unter Nutzung eines Sinkers gemäß der Japanischen Pharmakopöe durchgeführt wird.

8. Feste, oral applizierbare pharmazeutische Darreichungsform gemäß einem der Ansprüche 1 bis 7, **dadurch gekennzeichnet, dass** die Gesamtdosis des Wirkstoffs (I) 2,5 mg bis 30 mg beträgt.

9. Feste, oral applizierbare pharmazeutische Darreichungsform gemäß einem der Ansprüche 1 bis 8, **dadurch gekennzeichnet, dass** der Wirkstoff (I) in kristalliner Form enthalten ist.

10. Feste, oral applizierbare pharmazeutische Darreichungsform gemäß Anspruch 9, **dadurch gekennzeichnet, dass** der Wirkstoff (I) in mikronisierter Form enthalten ist.

11. Feste, oral applizierbare pharmazeutische Darreichungsform gemäß einem der Ansprüche 1 bis 10, **dadurch gekennzeichnet, dass** die Wirkstoffschicht des osmotischen Zweikammersystems Polyethylenoxid mit einer Viskosität von 40 bis 100 mPa*s (5%ige wässrige Lösung, 25°C) als osmotisch aktives Polymer enthält und die Osmoseschicht des osmotischen Zweikammersystems Polyethylenoxid mit einer Viskosität von 5000 bis 8000 mPa*s (1%ige wässrige Lösung, 25°C) als osmotisch aktives Polymer enthält.

12. Feste, oral applizierbare pharmazeutische Darreichungsform gemäß einem der Ansprüche 1 bis 11, **dadurch gekennzeichnet, dass** die Membranhülle des osmotischen Zweikammersystems aus Celluloseacetat oder einem Gemisch von Celluloseacetat und Polyethylenglycol besteht.

13. Verfahren zur Herstellung einer festen, oral applizierbaren pharmazeutische Darreichungsform gemäß einem der Ansprüche 1 bis 12, **dadurch gekennzeichnet, dass** die Komponenten der Wirkstoffschicht gemischt und vorzugsweise granuliert werden, die Komponenten der Osmoseschicht gemischt und vorzugsweise granuliert werden, anschließend beide Granulate auf einer Zweischichttablettenpresse zu einer Zweischichttablette verpresst werden und der so entstandene Kern dann mit einer semipermeablen Membran beschichtet und die Hülle auf der Wirkstoffseite mit einer oder mehreren Öffnungen versehen wird und anschließend der so entstandene membranlackierte Kern mit einer schnell freisetzenden Wirkstoffschicht umgeben wird, indem entweder ein den Wirkstoff (I) enthaltender Filmüberzug aufgetragen wird oder ein mittels Wirbelschichtgranulation hergestelltes wirkstoffhaltiges Granulat um den Kern gepresst wird.

14. Arzneimittel, enthaltend eine feste, oral applizierbare pharmazeutische Darreichungsform gemäß einem der Ansprüche 1 bis 12.

15. Oral applizierbare pharmazeutische Darreichungsform gemäß einem der Ansprüche 1 bis 12 zur Verwendung als Arzneimittel zur Prophylaxe, Sekundärprophylaxe und/oder Behandlung von thromboembolischen Erkrankungen.

## Claims

1. Solid orally administrable pharmaceutical dosage form comprising 5-chloro-*N*-({(5*S*)-2-oxo-3-[4-(3-oxo-4-morpholinyl)phenyl]-1,3-oxazolidin-5-yl}methyl)-2-thiophenecarboxamide (I), **characterized in that** it consists of a combination of rapid and controlled release, where
the active compound dose with controlled release is incorporated into an osmotic two-chamber system,
and
the osmotic release system is combined with an active compound-comprising film coating with rapid release of active compound (I) or an active compound-comprising mantle formed from powder or granules (core/mantle tablet).

2. Solid orally administrable pharmaceutical dosage form according to Claim 1, **characterized in that** 55 to 90% of active compound (I) are incorporated as controlled-release portion of the active compound dose into the osmotic two-chamber system and 10 to 45% of active compound (I) are incorporated as rapid-release portion of the active compound dose into the rapid-release film coating or an active-compound comprising mantle formed from powder or granules (core/mantle tablet).

3. Solid orally administrable pharmaceutical dosage form according to Claim 1 or 2, **characterized in that** 55 to 90% of the osmotic two-chamber system that comprises the active compound (I) consists of
A) an active compound layer having the composition
• 2 to 25% of active compound (I),
• 60 to 95% of one or more osmotically active polymers
B) an osmosis layer having the composition
• 40 to 90% of one or more osmotically active polymers,
• 10 to 40% of an osmotically active additive
and
C) a shell consisting of a material which is water-permeable but impermeable for the components of the core, which material has at least one opening on the active compound side.

4. Solid orally administrable pharmaceutical dosage form according to Claim 1 or 2, **characterized in that** the active compound-comprising film coating comprising 10 to 45% of active compound (I) is composed of 5 to 30% of active compound (I) and 0.1 to 2% of wetting agent, based on the dry weight of the film coatings.

5. Solid orally administrable pharmaceutical dosage form according to Claim 1 or 2, **characterized in that** the mantle formed from powder or granules (core/mantle tablet) and comprising 10 to 45% of active compound (I) is composed of 0.5 to 10% of active compound (I), based on the weight of the mantle formed from powder or granules.

6. Solid orally administrable pharmaceutical dosage form according to any of Claims 1 to 5, **characterized in that** the pharmaceutical dosage form releases 10 to 45% of active compound (I) of the declared total amount of active compound within 1 hour, 40 to 70% of active compound (I) within 4 hours and at least 80% of active compound (I) within 10 hours according to USP release method using apparatus 2 (paddle).

7. Solid orally administrable pharmaceutical dosage form according to Claim 6, **characterized in that** the USP-release method is carried out using apparatus 2 (paddle) at 75 rpm in 900 ml of a phosphate/citrate buffer of pH 6.8 with addition of 0.4% sodium lauryl sulphate as release medium and using a sinker according to the Japanese Pharmacopoeia.

8. Solid orally administrable pharmaceutical dosage form according to any of Claims 1 to 7, **characterized in that** the total dose of active compound (I) is 2.5 mg to 30 mg.

9. Solid orally administrable pharmaceutical dosage form according to any of Claims 1 to 8, **characterized in that** the active compound (I) is present in crystalline form.

10. Solid orally administrable pharmaceutical dosage form according to Claim 9, **characterized in that** the active compound (I) is present in micronized form.

11. Solid orally administrable pharmaceutical dosage form according to any of Claims 1 to 10, **characterized in that** the active compound layer of the osmotic two-chamber system comprises polyethylene oxide having a viscosity of 40 to 100 mPa*s (5% strength aqueous solution, 25°C) as osmotically active polymer and the osmosis layer of the osmotic two-chamber system comprises polyethylene oxide having a viscosity of 5000 to 8000 mPa*s (1% strength aqueous solution, 25°C) as osmotically active polymer.

12. Solid orally administrable pharmaceutical dosage form according to any of Claims 1 to 11, **characterized in that** the membrane shell of the osmotic two-chamber system consists of cellulose acetate or a mixture of cellulose acetate and polyethylene glycol.

13. Process for preparing a solid orally administrable pharmaceutical dosage form according to any of Claims 1 to 12, **characterized in that** the components of the active compound layer are mixed and preferably granulated, the components of the osmosis layer are mixed and preferably granulated, and the two granulates are then compressed on a bilayer tablet press to give a bilayer tablet and the resulting core is then coated with a semipermeable membrane and the shell is provided on the active compound side with one or more openings and the resulting membrane-coated core is then surrounded with a rapid-release active compound layer either by applying a film coating comprising the active compound (I) or by pressing active compound-comprising granules prepared by fluidized bed granulation onto the core.

14. Medicament, comprising a solid orally administrable pharmaceutical dosage form according to any of Claims 1 to 12.

15. Orally administrable pharmaceutical dosage form according to any of Claims 1 to 12 for use as medicament for the prophylaxis, secondary prophylaxis and/or treatment of thromboembolic disorders.

## Revendications

1. Forme pharmaceutique solide applicable par voie orale, contenant du 5-chloro-N-({(5S)-2-oxo-3-[4-(3-oxo-4-morpholinyl)-phényl]-1,3-oxazolidin-5-yl}-méthyl)-2-thiophène-carboxamide (I), **caractérisée en ce qu'**elle est constituée par une combinaison d'une libération rapide et contrôlée,
la dose d'agent actif à libération contrôlée étant incorporée dans un système osmotique à deux chambres, et
le système de libération osmotique étant combiné avec un revêtement de film contenant l'agent actif à libération rapide de l'agent actif (I) ou une enveloppe de poudre ou de granulat contenant l'agent actif (comprimé enveloppe-noyau).

2. Forme pharmaceutique solide applicable par voie orale selon la revendication 1, **caractérisée en ce que** 55 à 90 % de l'agent actif (I) est incorporé dans le système osmotique à deux chambres en tant que proportion à libération contrôlée de la dose d'agent actif, et 10 à 45 % de l'agent actif (I) est incorporé en tant que proportion à libération rapide de la dose d'agent actif dans le revêtement de film contenant l'agent actif à libération rapide ou une enveloppe de poudre ou de granulat contenant l'agent actif (comprimé enveloppe-noyau).

3. Forme pharmaceutique solide applicable par voie orale selon la revendication 1 ou 2, **caractérisée en ce que** le système osmotique à deux chambres contenant 55 à 90 % de l'agent actif (I) est constitué par :
A) une couche d'agent actif de composition
- 2 à 25 % d'agent actif (I),
- 60 à 95 % d'un ou de plusieurs polymères osmotiquement actifs,
B) une couche d'osmose de composition
- 40 à 90 % d'un ou de plusieurs polymères osmotiquement actifs,
- 10 à 40 % d'un additif osmotiquement actif,
et
C) une gaine, constituée par un matériau perméable à l'eau, mais toutefois imperméable aux composants du noyau, comprenant au moins une ouverture du côté de l' agent actif.

4. Forme pharmaceutique solide applicable par voie orale selon la revendication 1 ou 2, **caractérisée en ce que** le revêtement de film contenant l'agent actif qui contient 10 à 45 % de l'agent actif (I) est composé par 5 à 30 % de l'agent actif (I) et 0,1 à 2 % d'agents mouillants, par rapport au poids sec du revêtement de film.

5. Forme pharmaceutique solide applicable par voie orale selon la revendication 1 ou 2, **caractérisée en ce que** l'enveloppe de poudre ou de granulat contenant l'agent actif qui contient 10 à 45 % de l'agent actif (I) (comprimé enveloppe-noyau) est composée par 0,5 à 10 % d'agent actif (I), par rapport au poids de l'enveloppe de poudre ou de granulat.

6. Forme pharmaceutique solide applicable par voie orale selon l'une quelconque des revendications 1 à 5, **caractérisée en ce qu'**à partir de la forme pharmaceutique, 10 à 45 % de l'agent actif (I) de la quantité d'agent actif total déclarée est libérée après 1 heure, 40 à 70 % de l'agent actif (I) après 4 heures et au moins 80 % de l'agent actif (I) après 10 heures selon la méthode de libération USP avec l'appareil 2 (pale).

7. Forme pharmaceutique solide applicable par voie orale selon la revendication 6, **caractérisée en ce que** la méthode de libération USP avec l'appareil 2 (pale) est réalisée à 75 tours/minute dans 900 ml d'un tampon de phosphate-citrate de pH 6,8 avec un ajout de 0,4 % de laurylsulfate de sodium en tant que milieu de libération et en utilisant un support de capsule selon la pharmacopée japonaise.

8. Forme pharmaceutique solide applicable par voie orale selon l'une quelconque des revendications 1 à 7, **caractérisée en ce que** la dose totale de l'agent actif (I) est de 2,5 mg à 30 mg.

9. Forme pharmaceutique solide applicable par voie orale selon l'une quelconque des revendications 1 à 8, **caractérisée en ce que** l'agent actif (I) est contenu sous forme cristalline.

10. Forme pharmaceutique solide applicable par voie orale selon la revendication 9, **caractérisée en ce que** l'agent actif (I) est contenu sous forme micronisée.

11. Forme pharmaceutique solide applicable par voie orale selon l'une quelconque des revendications 1 à 10, **caractérisée en ce que** la couche d'agent actif du système osmotique à deux chambres contient du polyoxyde d'éthylène d'une viscosité de 40 à 100 mPa*s (solution aqueuse à 5 %, 25 °C) en tant que polymère osmotiquement actif, et la couche d'osmose du système osmotique à deux chambres contient du polyoxyde d'éthylène d'une viscosité de 5 000 à 8 000 mPa*s (solution aqueuse à 1 %, 25 °C) en tant que polymère osmotiquement actif.

12. Forme pharmaceutique solide applicable par voie orale selon l'une quelconque des revendications 1 à 11, **caractérisée en ce que** la gaine membranaire du système osmotique à deux chambres est constituée d'acétate de cellulose ou d'un mélange d'acétate de cellulose et de polyéthylène glycol.

13. Procédé de fabrication d'une forme pharmaceutique solide applicable par voie orale selon l'une quelconque des revendications 1 à 12, **caractérisé en ce que** les composants de la couche d'agent actif sont mélangés et de préférence granulés, les composants de la couche d'osmose sont mélangés et de préférence granulés, puis les deux granulats sont comprimés sur une presse à comprimés à deux couches pour former un comprimé à deux couches, et le noyau ainsi obtenu est ensuite revêtu avec une membrane semi-perméable, et la gaine sur le côté de l'agent actif est munie d'une ou de plusieurs ouvertures, puis le noyau revêtu de la membrane ainsi obtenu est entouré avec une couche d'agent actif à libération rapide, par application d'un revêtement de film contenant l'agent actif (I) ou compression d'un granulat contenant l'agent actif fabriqué par granulation en lit fluidisé autour du noyau.

14. Médicament, contenant une forme pharmaceutique solide applicable par voie orale selon l'une quelconque des revendications 1 à 12.

15. Forme pharmaceutique applicable par voie orale selon l'une quelconque des revendications 1 à 12, destinée à une utilisation en tant que médicament pour la prophylaxie, la prophylaxie secondaire et/ou le traitement de maladies thromboemboliques.
